# FASCICULE DE BREVET EUROPEEN

(11) **EP 0 583 459 B1**
(45) Date de publication et mention de la délivrance du brevet: **20.03.1996**
(21) Numéro de dépôt: 93905440.9
(22) Date de dépôt: 26.02.1993
(51) Int. Cl.: A61K 7/48, A61K 7/00, A61K 7/06, A61K 7/043

(54) **COMPOSITION COSMETIQUE OU DERMATOLOGIQUE SOUS LA FORME D'UNE DISPERSION HUILE-DANS-L'EAU SUSCEPTIBLE DE FORMER DES FILMS COMPOSITES**
KOSMETISCHE ODER DERMATOLOGISCHE ZUSAMMENSETZUNG IN FORM EINER ÖL-IN-WASSER DISPERSION, DIE VERBUNDFILME BILDEN KANN
COSMETIC OR DERMATOLOGICAL COMPOSITION IN THE FORM OF AN OIL-IN-WATER DISPERSION CAPABLE OF FORMING COMPOSITE FILMS

(30) Priorité: 27.02.1992 FR 9202296
(43) Date de publication de la demande: 23.02.1994
(73) Titulaire: L'OREAL, F-75008 Paris (FR)
(72) Inventeur: ARNAUD, Pascal, F-75009 Paris (FR); MELLUL, Myriam, F-94240 L'Hay-les-Roses (FR)
(74) Mandataire: Stalla-Bourdillon, Bernard
(86) Numéro de dépôt international: FR9300204
(87) Numéro de publication internationale: WO9316684

(56) Documents cités:
- EP-A- 0 196 904
- EP-A- 0 360 292
- EP-A- 0 390 206
- EP-A- 0 422 984
- WO-A-91/12793
- WO-A-93/11103
- DE-A- 2 052 579
- FR-A- 2 311 564
- GB-A- 2 190 393
- US-A- 4 059 688
- US-A- 4 514 537
- PATENT ABSTRACTS OF JAPAN vol. 012, no. 349 (C-529)20 Septembre 1988

## Description

La présente invention a pour objet une composition cosmétique ou dermatologique sous forme d'une dispersion huile-dans-l'eau susceptible de former des films composites sur certains substrats, notamment sur les matières kératiniques telles que la peau, les cheveux, les cils, les sourcils, les ongles ou les lèvres, agissant en tant qu'écran à l'égard de solvants tels que l'eau.

L'invention concerne plus particulièrement une composition cosmétique ou dermatologique sous forme d'une dispersion d'huile(s) fluorée(s) dans une solution aqueuse d'un polymère hydrosoluble tel que l'alcool polyvinylique et ses copolymères.

La préparation de films composites nécessite l'association de polymères filmogènes à des substances censées modifier les caractéristiques du film obtenu soit par association chimique soit par association physique (solubilisation ou dispersion).

Dans le cas d'une association chimique, le film composite est préparé par une réaction préalable de la substance à incorporer directement avec le polymère filmogène tel que décrit par exemple dans la demande de brevet français n° 76.15335 (n° de publication 2.311.564).

Dans le cas d'une association physique, on sait que l'on peut obtenir des films composites formés par séchage contenant certaines substances qui se retrouvent "solubilisées" dans le film au sens où elles sont solubilisées dans la phase aqueuse à partir de laquelle, par application sur un substrat, tel que les matières kératiniques, on obtient le film. Parmi ces substances on citera les plastifiants, les principes actifs et les colorants hydrosolubles.

On connait en outre des films composites contenant des substances non hydrosolubles qui y sont incorporées, également lors de la préparation du film, par dispersion dans la phase aqueuse avant que celle-ci ne soit appliquée sur un substrat tel que la peau. Parmi ces substances on citera les charges, les pigments ou des substances lipophiles telles que les huiles.

Les documents EP-A-0 390 206 et EP-A-0 196 904 décrivent des compositions comprenant de perfluoropolyethers.

Or, à la connaissance de la Société déposante, l'incorporation d'huiles fluorées sous forme dispersée dans une solution aqueuse de polymères hydrosolubles tels que l'alcool polyvinylique et ses copolymères en vue d'obtenir un film composite, n'a pas été décrite jusqu'à présent.

Il existe toutefois des dispersions d'huiles fluorées dans des solutions aqueuses de polymères hydrosolubles.On citera à cet égard la demande de brevet européen n° 360.292 qui décrit des émulsions destinées à nettoyer la peau mais qui n'ont pas la propriété de former des films protecteurs.

La Société déposante a en effet pu mettre en évidence que l'élimination de l'eau des émulsions décrites dans cette demande de brevet, par évaporation à température ambiante, ne permet pas de conduire à la formation d'un film homogène.

Après diverses études, on a constaté que suite à un choix particulier d'une classe de polymères filmogènes, on pouvait obtenir d'une manière inattendue et surprenante, une composition sous forme d'une dispersion huile-dans'l'eau conduisant à des films composites dans lesquels les substances à incorporer, à savoir des huiles fluorées, restent parfaitement dispersées à l'intérieur du film composite une fois obtenu.

En effet, on a découvert que le choix de l'alcool polyvinylique et de certains de ses copolymères conduisait à des compositions très satisfaisantes en ce qui concerne la qualité et la stabilité des films obtenus.

La présente invention a donc pour objet une composition cosmétique ou dermatologique sous forme d'une dispersion huile-dans-l'eau susceptible de former un film composite par évaporation de la phase aqueuse continue caractérisée par le fait qu'elle comprend au moins une huile fluorée dispersée dans une solution aqueuse d'au moins un polymère hydrosoluble comprenant de 50 à 100 % en moles de motifs de formule suivante : 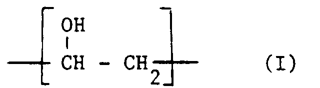
Ce polymère peut donc être soit l'alcool polyvinylique soit l'un de ses copolymères.

Grâce au choix de ce type de polymère, il est maintenant possible d'obtenir des films composites contenant des huiles fluorées et ayant des caractéristiques intéressantes telles qu'une augmentation de la souplesse et de l'élasticité.

On a pu par ailleurs mettre en évidence, que les films obtenus à partir de la composition selon l'invention, présentaient une diminution de la sensibilité à l'eau ce qui les rend moins poisseux à son contact en diminuant leur vitesse de redissolution.

En outre, on a remarqué que les films obtenus à partir de la composition selon l'invention, possèdaient de bonnes propriétés sensorielles tactiles, la présence des huiles fluorées conduisant à un toucher plus doux et plus agréable du film.

Les compositions cosmétiques ou dermatologiques selon l'invention peuvent se présenter sous forme de masques de beauté, de gels amincissants, de crèmes protectrices, de gels coiffants, d'eye-liners, de mascaras éventuellement colorés, de vernis à ongles aqueux, de laques à l'eau, de gels de protection labiale ou de produits de coloration temporaire des cheveux.

Lorsque l'on utilise selon l'invention de l'alcool polyvinylique, celui-ci est obtenu par hydrolyse alcaline du polyacétate de vinyle. On considère qu'un alcool polyvinylique issu du polyacétate de vinyle est totalement hydrolysé lorsque le taux d'hydrolyse est égal ou supérieur à 98 % en moles.

Lorsque le polymère utilisé selon l'invention est un copolymère de l'alcool polyvinylique, il comprend de 1 à 50 % en moles de motifs répondant à la formule suivante : 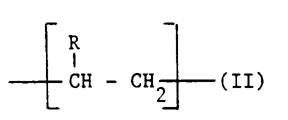
dans laquelle
R est un atome d'hydrogène, un atome de chlore, un atome de fluor ou représente le groupement 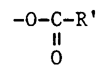
ou le groupement OR',
R' représentant un radical alkyle ayant de préférence de 1 à 6 atomes de carbone.

Le polymère hydrosoluble utilisé selon l'invention est présent de manière générale à raison de 0,5 à 40 % et de préférence de 0,75 à 20 % en poids par rapport au poids total de la composition.

L'alcool polyvinylique et ses copolymères utilisés selon l'invention ont une masse moléculaire comprise de préférence entre 15.000 et 250.000, et plus particulièrement entre 25.000 et 200.000.

Parmi les alcools polyvinyliques et les copolymères d'alcool polyvinylique/acétate de vinyle, on peut citer notamment :
- les produits vendus sous la dénomination de "RHODOVIOL" par la Société RHONE POULENC, et en particulier :
   - le "RHODOVIOL 25/140" qui est alcool polyvinylique partiellement acétylé (degré d'hydrolyse : 89 % ; PM = 103.000), et
   - le "RHODOVIOL 4/125p" qui est copolymère d'alcool polyvinylique/acétate de vinyle, ainsi que
   - le "RHODOVIOL 25/145".
- les produits vendus sous la dénomination de "MOWIOL" par la Société HOECHST, et en particulier :
   - le "MOWIOL 40-88" qui est un alcool polyvinylique partiellement acétylé (degré d'hydrolyse : 87,7 %, PM = 127.000)
   - le "MOWIOL 18-88" qui est un alcool polyvinylique partiellement acétylé (degré d'hydrolyse : 87,7 %, PM = 84.000)
- les produits vendus sous la dénomination de "POLYVIOL" par la Société WACKER,
- les produits vendus sous la dénomination de "POVAL" par la Société KURARAY, et
- les produits vendus sous la dénomination de "AIRVOL" par la Société AIR PRODUCTS.

Parmi les copolymères d'alcool polyvinylique/éthylène, on peut citer les produits vendus sous la dénomination de "CLARENE" par la Société SOLVAY.

Les copolymères d'alcool polyvinylique dans lesquels le radical R de la formule II représente un atome de fluor ou OR' sont décrits dans les brevets US 2.499.097 et EP 140.325.

Selon un mode de réalisation préféré, la composition sous forme de dispersion selon l'invention peut comporter de 0,01 à 50 % et de préférence de 0,05 à 40 % par rapport au poids total de la composition d'au moins un autre polymère filmogène hydrosoluble choisi notamment parmi :
- les dérivés de kératine, tels que les hydrolysats de kératine et les kératines sulfoniques,
- les dérivés de chitine ou de chitosane anioniques, cationiques, amphotères ou non ioniques,
- les dérivés de cellulose, tels que l'hydroxyéthylcellulose, l'hydroxypropylcellulose, la méthylcellulose, l'hydroxypropylméthylcellulose, l'éthylhydroxyéthylcellulose, la carboxyméthylcellulose, ainsi que les dérivés quaternisés de la cellulose,
- les polymères acryliques, tels que les polyacrylates et les polyméthacrylates, ainsi que les copolymères acryliques,
- la polyvinylpyrrolidone et les copolymères vinyliques, autres que ceux indiqués ci-dessus, tels que le copolymère de l'éther méthylvinylique et de l'anhydride maléique éventuellement monoestérifié ou monoamidifié, ou le copolymère de l'acétate de vinyle et de l'acide crotonique, et
- les polymères naturels tels que les gommes arabiques, la gomme de guar, les dérivés du xanthane et la gomme de karaya, les alginates et les carraghénates, les glycoaminoglycanes, l'acide hyaluronique et ses dérivés.

Ces polymères filmogènes hydrosolubles additionnels constituent avec le polymère hydrosoluble à motifs de formule (I) utilisé selon l'invention, la matrice du film composite dans laquelle se retrouve à l'état de dispersion la ou les huiles fluorées.

Selon l'invention, il est préférable d'utiliser comme huile fluorée soit une huile carbonée, éventuellement fonctionnalisée, dont les atomes d'hydrogène ont été partiellement ou totalement remplacés par des atomes de fluor, soit une silicone fluorée.

Par l'emploi du terme "fonctionnalisé", on entend une substitution de la chaîne par divers groupements organiques.

Parmi les huiles carbonées ainsi définies on peut citer les composés organofluorés hydrocarbonés partiellement fluorés et perfluorés, les perfluoropolyéthers fonctionnalisés ou non fonctionnalisés et les polyéthers fluorohydrocarbonés et leurs mélanges.

Parmi les composés organofluorés hydrocarbonés perfluorés utilisables selon l'invention, on peut citer :
1) Ceux appartenant au groupe des perfluoroalcanes, des perfluorocycloalcanes, des perfluoropolycycloalcanes et des perfluoro(alkylcycloalcanes).
   Comme exemples de perfluoroalcanes, on peut citer la série des alcanes linéaires tels que le perfluorooctane, le perfluorononane ou encore le perfluorodécane.
   Comme exemples de perfluorocycloalcanes et de perfluoro (alkylcyloalcanes), on peut citer la perfluorodécaline vendue par la Société RHONE POULENC sous la dénomination de "FLUTEC PP5", la perfluoro(méthyldécaline), les perfluoro (C₃-C₅-alkylcyclohexanes) tels que le perfluoro(butylcyclohexane).
   Comme exemple de perfluoropolycycloalcanes, on peut citer les dérivés du bicyclo [3,3,1] nonane tels que le triméthylbicyclo [3,3,1] nonane, les dérivés de l'adamantane tel que le diméthyladamantane et les dérivés perfluorés de phénanthrène hydrogéné tels que le tétracosafluoro- tetradécahydro phénanthrène.
2) Ceux appartenant au groupe des hydrocarbures perfluorés aromatiques (perfluoroarènes) comme les dérivés perfluorés du naphtalène tels que le perfluoronaphtalène et le perfluorométhyl-1-naphtalène.
3) Ceux appartenant au groupe des composés organo perfluorés hydrocarbonés contenant au moins un hétéroatome, par exemple les amines tertiaires perfluorées comme la perfluorotributylamine, ou des composés hétérocycliques saturés, substitués par des groupements alkyles, comme les perfluoro(alkyl tétrahydropyrannes) tels que la perfluoro(hexyltétrahydropyranne), les perfluro (alkyltétrahydrofurannes) tels que le perfluoro(heptyltétrahydrofuranne) et le perfluoro(butyltétrahydrofuranne) ou les dérivés de la morpholine comme la perfluoro(N-pentylmorpholine).

Parmi les composés organofluorés hydrocarbonés partiellement fluorés on peut citer ceux de formule suivante :

(RF)ₓ-(A)_{y}-(RH)_{z} (III)

dans laquelle :
x peut être 1,2 ou 3 ;
z peut être 0, 1, 2 ou 3 ;
y peut être 0 ou 1 ;
z et y ne pouvant être simultanément zéro, et
lorsque z = 0, alors y = 1 et x = 2 ou 3.

RF représente un radical fluoré aromatique ou aliphatique, saturé ou insaturé, dont la chaîne peut être linéaire, ramifiée ou cyclique, de préférence un radical perfluoroalkyle ayant de 6 à 20 atomes de carbone, la chaîne pouvant éventuellement être fonctionnalisée et/ou pouvant être interrompue par des atomes divalents tels que l'oxygène ou le soufre, ou trivalents comme l'azote, la chaîne pouvant en outre être substituée par des atomes d'hydrogène ou d'autres halogènes, à condition que pas plus d'un de ces substituants, autres que le fluor, ne soit présent pour 2 atomes de carbone,
RH représente un radical hydrocarboné aliphatique ou aromatique, saturé ou insaturé, dont la chaîne peut être linéaire, ramifiée ou cyclique éventuellement fonctionnalisée et/ou interrompue par des atomes divalents tels que l'oxygène ou le soufre, ou par des atomes trivalents comme l'azote, et A représente un radical di, tri ou quadrivalent tel que :
〉C〈 , 〉CH〈 , -N〈 , -CO-N〈 , -SO₂N〈, 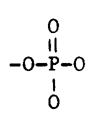
les structures cycliques, aliphatiques ou aromatiques ou les insaturations éthyléniques.

Par fonctionnalisé, on entend selon l'invention, une substitution du squelette intercalaire, terminale ou pendante, par au moins un groupement organique fonctionnel comme une fonction alcool, thiol, acide, carbonyle, sulfoxyde, ester, amide, amine, phosphate.

Par insaturation éthylénique, on entend par exemple :
〉C=C〈 , 〉C=CH- ou -CH=CH-

De préférence, RH représente un radical alkyle linéaire ou ramifié en C₁-C₂₂ ou un mélange de radicaux alkyles linéaires ou ramifiés en C₁-C₂₂, un radical aryle en C₆-C₁₀ ou un radical aralkyle en C₇-C₁₅.

De préférence, RF représente un radical perfluoroalkyle ayant de 4 à 22 atomes de carbone.

A titre d'exemple de composés organofluorés hydrocarbonés partiellement fluorés utilisables selon l'invention, on peut citer les produits ayant la formule suivante : 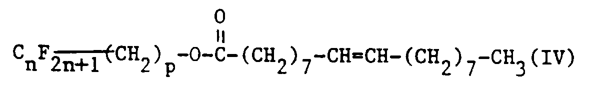
dans laquelle :
n est un nombre entier égal à 6 ou 8 et p est 1 ou 2, vendus sous les dénominations de "NOFABLE FO" par la Société "NIPPON OIL & FATS CO".

On peut citer aussi les composés possédant des groupes perfluorocarbures et des groupes hydrocarbures, le nombre total de carbone étant compris entre 10 et 30, le nombre d'atomes de carbone des groupes hydrocarbures étant égal ou supérieur à deux fois le nombre d'atomes de carbone des groupes perfluorocarbures, tels qu'ils sont décrits dans le document JP 63-002916.

De même, à titre illustratif, on peut citer les fluorohydrocarbures décrits dans la demande de brevet FR 9115019 et dont la structure générale est définie par la formule (V).

R_{f}-(CH₂)ₙ-X-[C₃H₅(OH)]-(O)ₓ-Rₕ (V)

où [C₃H₅(OH)] représente 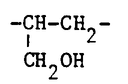
ou -CH₂-CHOH-CH₂-
R_{f} représente un radical alkyle perfluoré en C₄-C₂₀ ou un mélange de radicaux perfluorés en C₄-C₂₀,
Rₕ représente un radical alkyle linéaire ou ramifié en C₁-C₂₂ ou un mélange de radicaux alkyles linéaires ou ramifiés en
C₁-C₂₂ ou un radical aryle en C₆-C₁₀ ou aralkyle en
C₇-C₁₅,
n est compris entre O et 4,
X représente un atome de soufre ou d'oxygène, et
x représente O ou 1.

Les composés peuvent être préparés par réaction du composé fluoré correspondant R_{f}-(CH₂)ₙ-X-H avec l'époxyde correspondant 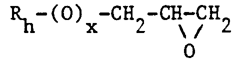
en présence d'un composé basique ou acide qui joue le rôle de réactif ou de catalyseur. Parmi ceux-ci, on peut citer le 1-(2'-F-hexyléthylthio)-3-(2''-éthylhéxyloxy)2-propanol.

Parmi les perfluoropolyéthers non fonctionnalisés, on peut citer :
1) Ceux ayant la formule suivante : 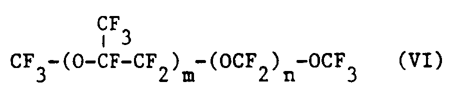 dans laquelle :
   m/n = 5 à 40, le poids moléculaire moyen étant supérieur à 500 et de préférence compris entre 1000 et 10 000.
   Parmi ceux-ci, on peut citer ceux vendus par la Société MONTEFLUOS sous les dénominations "GALDEN", "FOMBLIN Y", "FOMBLIN HC" et notamment le "FOMBLIN HC25" qui est un perfluoropolyméthylisopropyléther de formule: 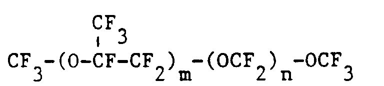 où m/n = 20 à 40 ; PM = 3.200.
2) Ceux ayant la formule suivante :

   CF₃-(O-CF₂-CF₂)ₚ-(OCF₂)_{q}-OCF₃ (VII)

   dans laquelle :
   p/q est de 0,5 à 1,5, le poids moléculaire moyen étant supérieur à 500 et de préférence compris entre 1000 et 10.000.
   Parmi ceux-ci, on peut citer celui vendu sous la dénomination de "FOMBLIN Z" par la Société MONTEFLUOS ;
3) Ceux ayant la formule suivante : 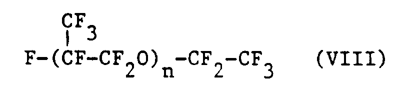 dans laquelle :
   n est un nombre entier de 4 à 500.
   Parmi ceux-ci on peut citer celui vendu sous la dénomination de "KRYTOX" par la Société DUPONT DE NEMOURS.
4) Ceux ayant la formule suivante : 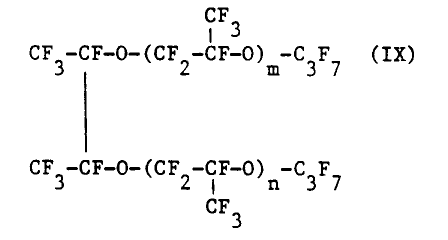 dans laquelle :
   n et m sont des nombres entiers de 0 à 3.

Parmi ceux-ci, on peut citer celui vendu sous la dénomination "HOSTINERT" par la Société HOECHST.

Parmi les perfluoropolyéthers fonctionnalisés on peut citer ceux ayant la formule suivante :

RCF₂-(O-CF₂CF₂)ₚ-(OCF₂)_{q}- OCF₂R (X)

dans laquelle :
p/q est de 0,5 à 1,5 et
R représente un reste -COOCH₃, -CH₂OH,
-CH₂O-CH₂-CHOH-CH₂OH ou -CH₂-(OCH₂-CH₂)ₜ-OH où t est 1 ou 2, le poids moléculaire moyen étant supérieur à 500 et de préférence compris entre 1.000 et 10.000.

Parmi ceux-ci on peut citer ceux vendus par la Société MONTEFLUOS sous les dénominations de :
- "FOMBLIN Z-DOL" (R = -CH₂OH : PM = 2.000)
- "FOMBLIN Z-tetraol" (R = -CH₂OH-CHOH-CH₂OH ; PM = 1.900), et
- "FOMBLIN Z-DOL-TX" (R = -CH₂(OCH₂CH₂)ₜOH, t étant 1 ou 2) par la Société MONTEFLUOS.

Parmi les silicones fluorées on peut citer celles ayant la formule (XI) suivante : 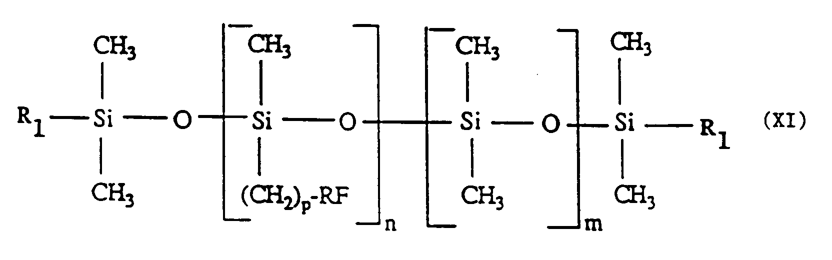
dans laquelle :
R₁ représente : -CH₃, -OH ou (̵CH₂)̵ₚRF,
n est un nombre entier de 1 à 300,
m est un nombre entier de 0 à 150,
p est un nombre entier de 0 à 6 et
RF est un radical perfluoroalkyle ayant de 1 à 9 atomes de carbone.

Parmi les silicones fluorées on peut citer celles vendues par la Société Shin-Etsu, sous les dénominations de "FL-100", "X 22819", "X 22820" "X 22821" et "X 22822", ainsi que celle vendue par la Société DOW CORNING sous la dénomination de "ES 1265" et celle vendue par la Société GENERAL ELECTRIC sous la dénomination "FF 150".

Selon l'invention, l'huile fluorée est présente à raison de 0,01 à 90 % en poids et de préférence entre 0,1 à 50 % en poids par rapport au poids total de la composition avec de préférence un rapport en poids de polymère hydrosoluble à motifs de formule (I) à l'huile fluorée compris entre 0,01 et 500.

Les compositions selon l'invention peuvent contenir dans la phase aqueuse au moins un additif communément utilisé en cosmétique ou en dermatologie, tel que par exemple un actif hydrosoluble tel qu'un agent hydratant, en particulier un composé polyhydroxylé.

On citera par exemple le glycérol, le sorbitol ou le D-panthénol qui sont également utiles pour plastifier les films obtenus par étalement des compositions cosmétiques selon l'invention. Parmi d'autres additifs, on peut citer des agents amincissants, des agents anti-rides, des stimulants, des revitalisants, des raffermissants et des adoucissants. Les additifs sont présents de préférence en une proportion comprise entre 0,01 et 30 % en poids par rapport au poids total de la composition.

Les compositions cosmétiques et dermatologiques selon l'invention peuvent également contenir dans la phase aqueuse des épaississants tels que par exemple du CARBOPOL (vendu par la Société GOODRICH), des huiles émulsionnées, des polymères non filmogènes, des polymères filmogènes autres que ceux cités ci-dessus, des conservateurs, des séquestrants, des agents anti-mousse, des parfums, des colorants, des pigments, des modificateurs de pH ou encore des charges.

Les compositions selon l'invention, peuvent être obtenues de la manière suivante :
On prépare dans un premier temps une solution aqueuse dans laquelle le polymère hydrosoluble à motifs de formule (I) ainsi qu'éventuellement un polymère filmogène additionnel est (ou sont) solubilisé(s) en une proportion telle que la viscosité ne dépasse pas approximativement 10.000 mPa.s. Si désiré, des additifs tels que ceux mentionnés ci-dessus ou bien d'autres peuvent être ajoutés à ce stade de la préparation.

A partir de la solution aqueuse ainsi préparée, on disperse alors, sous vive agitation, l'huile fluorée, seule ou en mélange, de manière classique en une proportion telle qu'indiquée ci-dessus en utilisant par exemple un agitateur de type Moritz ou Rayneri à une température proche de la température ambiante.

La taille des particules ainsi obtenues dans la composition selon l'invention peut varier entre 0,2 et 50 µm, de préférence entre 0,5 et 10 µm.

La composition filmogène selon l'invention peut être appliquée sur un support kératinique adapté tel que la peau, les cheveux, les cils, les sourcils, les lèvres et les ongles sous forme d'une couche homogène de faible épaisseur puis on laisse évaporer l'eau de la phase continue à température ambiante afin d'obtenir un film composite. En l'absence de matière colorante et de charge, le film ainsi formé apparaît translucide à opaque suivant le pourcentage d'huile(s) fluorée(s) qu'il renferme. Sa surface est parfaitement homogène à l'état sec, et ne colle pas. On observe après séchage du film qu'aucune démixtion de l'huile fluorée ne s'est produite et que celle-ci reste dispersée dans le film sous la forme de gouttelettes qui de manière surprenante, ont un diamètre sensiblement égal à celui des particules de la dispersion aqueuse avant séchage, à savoir de 0,2 à 50 µm.

Le temps d'évaporation et de séchage du film obtenu à partir des compositions filmogènes selon l'invention dépend de la nature des compositions mais est de l'ordre de 1 à 15 minutes et de préférence de 2 à 10 minutes.

Selon un mode de réalisation préféré, la formation du film peut être accélérée par addition, à la composition sous forme de dispersion, d'un alcool de bas poids moléculaire tel que l'éthanol ou l'isopropanol en une proportion comprise entre 0,01 et 20 %.

Les exemples qui suivent illustrent l'invention sans pour autant la limiter.

### EXEMPLES DE COMPOSITIONS FILMOGENES COSMETIQUES ET DERMATOLOGIQUES

On prépare ces compositions par la dispersion préalable d'huile(s) fluorée(s) dans une solution aqueuse de (ou des) polymère(s) polyhydroxylé(s), de préférence à une température proche de la température ambiante. Ensuite, on mélange cette dispersion avec les autres constituants présents dans la formule.

### EXEMPLE A : Gel coiffant

| | |
|---|---|
| - "CARBOPOL 940" | 0,25 g |
| - Triéthanolamine qs pH = 6,5 | |
| - Copolymère polyvinylpyrrolidone/acétate de vinyle vendu par la Société GAF | 1,00 g |
| - Polymère hydrosoluble"MOWIOL 40-88" | 1,00 g |
| - Huile fluorée "FOMBLIN HC25" | 1,00 g |
| - Alcool éthylique | 10,00 g |
| - Conservateur qs | |
| - Eau qsp | 100,00 g |

Appliquée sur les cheveux, la composition forme un film qui permet un maintien naturel de la coiffure.

### EXEMPLE B : Gel traitant pour les cils

| | |
|---|---|
| - "CARBOPOL 940" | 1,40 g |
| - Triéthanolamine qs pH = 6,5 | |
| - Copolymère hydrosoluble d'alcool polyvinylique/acétate de vinyle vendu sous la dénomination de "RHODOVIOL 4/125 P" par la Société RHONE POULENC | 2,30 g |
| - Huile fluorée "FOMBLIN HC25" | 1,00 g |
| - Glycérol | 1,00 g |
| - D-Panthénol | 5,00 g |
| - Conservateur qs | |
| - Eau qsp | 100,00 g |

Par application sur les cils, ce gel sèche rapidement et forme un film hydratant.

### EXEMPLE C : Eye-liner

| | |
|---|---|
| - "CARBOPOL 941" | 0,20 g |
| - Triéthanolamine qs pH = 6,5 | |
| - Hydroxy-éthylcellulose | 0,30 g |
| - Polymère hydrosoluble "MOWIOL 18-88" | 1,00 g |
| - Huile fluorée "FOMBLIN HC25" | 5,00 g |
| - Glycérol | 3,00 g |
| - Oxyde de fer noir | 10,00 g |
| - Conservateur qs | |
| - Eau qsp | 100,00 g |

Appliqué sur les paupières, au ras des cils, l'eye-liner sèche rapidement et forme un trait homogène.

### EXEMPLE D : Masque pour le visage

| | |
|---|---|
| - Bentonite | 1,80 g |
| - Polymère hydrosoluble "RHODOVIOL 25/140" | 8,90 g |
| - Polymère hydrosoluble "RHODOVIOL 4/125 P" | 8,90 g |
| - Huile fluorée "FOMBLIN HC25" | 10,00 g |
| - Dioxyde de titane | 0,90 g |
| - Oxyde de fer | 0,05 g |
| - Alcool éthylique | 5,00 g |
| - Conservateur qs | |
| - Parfum qs | |
| - Eau qsp | 100,00 g |

Par application sur le visage, ce masque forme un film pelliculable après environ 5 minutes.

### EXEMPLE E : Gel amincissantt

| | |
|---|---|
| - Polymère hydrosoluble "RHODOVIOL 25/140" | 12,00 g |
| - Huile fluorée "FOMBLIN HC 25" | 7,50 g |
| - Huile fluorée "FOMBLIN Z-TETRAOL" | 2,50 g |
| - Hydroxypropyl méthyl cellulose | 0,05 g |
| - Caféine | 3,00 g |
| - Conservateur qs | |
| - Eau qsp | 100,00 g |

Par application sur la peau à l'endroit à traiter, il se forme un film pelliculable après 10 minutes.

### EXEMPLE F : Masque pour le visage

| | |
|---|---|
| - Bentonite | 1,8 g |
| - Polymère hydrosoluble "RHODOVIOL 4/125 P" | 17,8 g |
| - Huile fluorée "FOMBLIN HC25" | 0,5 g |
| - Dioxyde de titane | 0,9 g |
| - Oxyde de fer | 0,05 g |
| - Alcool éthylique | 5,0 g |
| - Conservateur qs | |
| - Parfum qs | |
| - Eau qsp | 100,0 g |

Par application sur le visage, ce masque forme un film pelliculable après environ 5 minutes.

### EXEMPLE G : Masque

| | |
|---|---|
| - Bentonite | 1,80 g |
| - Polymère hydrosoluble "RHODOVIOL 25/140" | 8,90 g |
| - Polymère hydrosoluble "RHODOVIOL 4/125 P" | 8,90 g |
| - Huile fluorée FLUTEC PP5 | 5,00 g |
| - Dioxyde de titane | 0,90 g |
| - Oxydes de fer | 0,05 g |
| - Alcool éthylique | 5,00 g |
| - Conservateur qs | |
| - Parfum qs | |
| - Eau qsp | 100,00 g |

Par application sur le visage, ce masque forme un film pelliculable après environ 5 minutes.

### EXEMPLE H : Gel amincissant

| | |
|---|---|
| - Polymère hydrosoluble "RHODOVIOL 25/140" | 12,00 g |
| - Hydroxypropylméthylcellulose vendue sous la dénomination "METHOCEL F4M STANDARD" vendu par la Société Dow Chemical | 0,05 g |
| - Huile fluorée "FLUTEC PP5" | 5,00 g |
| - Caféine | 3,00 g |
| - Conservateurs qs | |
| - Eau qsp | 100,00 g |

Par application sur la peau à l'endroit à traiter, il se forme un film pelliculable après 10 minutes.

### EXEMPLE I : Eye-liner

| | |
|---|---|
| - CARBOPOL 941 | 0,20 g |
| - Triéthénolamine qs pH = 6,5 | |
| - Hydroxy-éthylcellulose | 0,30 g |
| - Polymère hydrosoluble "MOWIOL 18-88" | 1,00 g |
| - Huile fluorée 1-(2'-F-hexyléthiolthio)3-(2''-ethylhexyloxy)-2 propanol | 3,00 g |
| - Glycérol | 3,00 g |
| - Oxyde de fer noir | 10,00 g |
| - Conservateur qs | |
| - Eau qsp | 100,00 g |

Appliqué sur les paupières, au ras des cils, l'eye-liner forme un film homogène, séchant rapidement, sous la forme d'un trait régulier.

### EXEMPLE J - Gel de protection labiale

| | |
|---|---|
| - Polymère hydrosoluble "RHODOVIOL 25/145" | 12 % |
| - Hydroxypropylmethylcellulose vendue sous la dénomination "METHOCEL FGM STANDARD" par la Société Dow Chemical | 3 % |
| - Huile fluorée "FOMBLIN HC25" | 0,5 % |
| - p-panthénol | 1 % |
| - Glycérol | 3 % |
| - Alcool éthylique | 1 % |
| - Conservateurs qs | |
| - Parfum qs | |
| - Eau qsp | 100 % |

Par application sur les lèvres, ce gel sèche rapidement et forme un film protecteur.

## Revendications

1. Composition cosmétique ou dermatologique sous forme d'une dispersion huile-dans-l'eau susceptible de donner un film composite par évaporation de la phase aqueuse continue caractérisée par le fait qu'elle comprend au moins une huile fluorée dispersée dans une solution aqueuse d'au moins un polymère hydrosoluble comprenant de 50 à 100 % en moles de motifs de formule suivante :

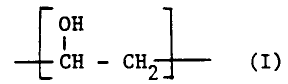

2. Composition selon la revendication 1 caractérisée par le fait que le polymère hydrosoluble comprend également de 1 à 50 % en moles de motifs de formule suivante : 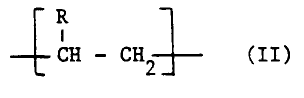 dans laquelle :
R est un atome d'hydrogène, un atome de chlore, un atome de fluor ou représente le groupement 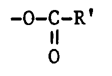 ou le groupement OR',
R' représentant un radical alkyle ayant de préférence de 1 à 6 atomes de carbone.

3. Composition selon la revendication 1 ou 2, caractérisée par le fait que le polymère hydrosoluble a un poids moléculaire compris entre 15.000 et 250.000, de préférence entre 25.000 et 200.000.

4. Composition selon l'une quelconque des revendications précédentes caractérisée par le fait que le polymère hydrosoluble est choisi parmi : l'alcool polyvinylique, les copolymères d'alcool polyvinylique/acétate de vinyle et d'alcool polyvinylique/éthylène.

5. Composition selon l'une quelconque des revendications précédentes caractérisée par le fait que le polymère hydrosoluble est présent à raison de 0,5 à 40 % et de préférence de 0,75 à 20 % en poids par rapport au poids total de la composition.

6. Composition selon l'une quelconque des revendications précédentes caractérisée par le fait que l'huile fluorée est soit une huile carbonée, éventuellement fonctionnalisée, dont les atomes d'hydrogène ont été partiellement ou totalement remplacés par des atomes de fluor, soit une silicone fluorée.

7. Composition selon la revendication 6, caractérisée par le fait que l'huile fluorée est choisie parmi les composés organofluorés hydrocarbonés partiellement fluorés et perfluorés, les perfluoropolyéthers et les polyéthers fluorohydrocarbonés.

8. Composition selon la revendication 7, caractérisée par le fait que les composés organofluorés hydrocarbonés perfluorés sont choisis parmi les perfluoroalcanes, les perfluorocycloalcanes, les perfluoropolycycloalcanes, les perfluoro(alkylcycloalcanes), les perfluoroarènes et les hydrocarbures perfluorés comportant au moins un hétéroatome.

9. Composition selon la revendication 7, caractérisée par le fait que les composés organofluorés hydrocarbonés partiellement fluorés répondent à la formule suivante :
(RF)ₓ-(A)_{y}-(RH)_{z} (III)
dans laquelle :
x peut être 1,2 ou 3 ;
z peut être 0, 1, 2, ou 3 ;
y peut être 0 ou 1 ;
z et y ne pouvant être simultanément zéro, et
lorsque z = 0, alors y = 1 et x = 2 ou 3 ;
RF représente un radical fluoré aromatique ou aliphatique, saturé ou insaturé, dont la chaîne peut être linéaire, ramifiée ou cyclique, de préférence un radical perfluoroalkyle ayant de 6 à 20 atomes de carbone, la chaîne pouvant éventuellement être fonctionnalisée et/ou pouvant être interrompue par des atomes divalents tels que l'oxygène ou le soufre, ou trivalents comme l'azote, la chaîne pouvant en outre être substituée par des atomes d'hydrogène ou d'autres halogènes, à condition que pas plus d'un de ces substituants, autres que le fluor, ne soit présent pour 2 atomes de carbone,
RH représente un résidu hydrocarboné aliphatique ou aromatique, saturé ou insaturé, dont la chaîne peut être linéaire, ramifiée ou cyclique éventuellement fonctionnalisée et/ou interrompue par des atomes divalents tels que l'oxygène ou le soufre, ou par des atomes trivalents comme l'azote,
et A représente une liaison covalente quand y = 0, et quand y est différent de 0, A représente un radical di- ou polyvalent.

10. Composition selon la revendication 9, caractérisée par le fait que lesdits composés organofluorés hydrocarbonés partiellement fluorés répondent à la formule suivante : 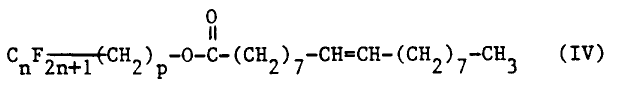 dans laquelle :
n est un nombre égal à 6 ou 8 et
p est 1 ou 2.

11. Composition selon la revendication 9, caractérisée par le fait que lesdits composés organofluorés hydrocarbonés partiellement fluorés répondent à la formule suivante :
R_{f}-(CH₂)ₙ-X-[C₃H₅(OH)]-(O)ₓ-Rₕ (V)
où [C₃H₅(OH)] représente 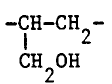 ou -CH₂-CHOH-CH₂-R_{f} représente un radical alkyle perfluoré en C₄-C₂₀ ou un mélange de radicaux perfluorés en C₄-C₂₀,
Rₕ représente un radical alkyle linéaire ou ramifié en C₁-C₂₂ ou un mélange de radicaux alkyle linéaires ou ramifiés en C₁-C₂₂ ou un radical aryle en C₆-C₁₀ ou aralkyle en C₇-C₁₅,
n est compris entre O et 4,
X représente un atome de soufre ou d'oxygène, et
x représente O ou 1.

12. Composition selon la revendication 7 caractérisée par le fait que les perfluoropolyéthers non fonctionalisés sont choisis parmi les formules suivantes :
(i) 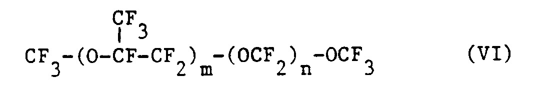 dans laquelle : m/n varie de 5 à 40, le poids moléculaire moyen étant supérieur à 500,
(ii)
CF₃-(O-CF₂CF₂)ₚ-(OCF₂)_{q}-OCF₃ (VII)
dans laquelle : p/q est de 0,5 à 1,5, le poids moléculaire moyen étant supérieur à 500,
(iii) 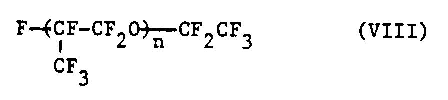 dans laquelle : n est un nombre entier de 4 à 500, et
(iv) 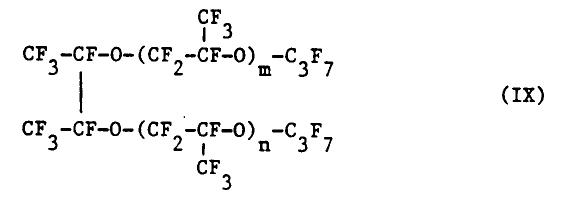 dans laquelle : n et m sont des nombres entiers de 0 à 3, ou un de leurs mélanges.

13. Composition selon la revendication 7, caractérisée par le fait que lesdits perfluoropolyéthers fonctionnalisés répondent à la formule suivante :
RCF₂-(OCF₂CF₂)ₚ-(OCF₂)_{q}- OCF₂R (X)
dans laquelle : p/q varie de 0,5 à 1,5 et
R est un reste -COOCH₃, -CH₂OH, -CH₂O-CH₂-CHOH-CH₂OH ou -CH₂-(OCH₂-CH₂)ₜ-OH où t est 1 ou 2, le poids moléculaire moyen étant supérieur à 500.

14. Composition selon la revendication 6 caractérisée par le fait que la silicone fluorée répond à la formule (XI) : 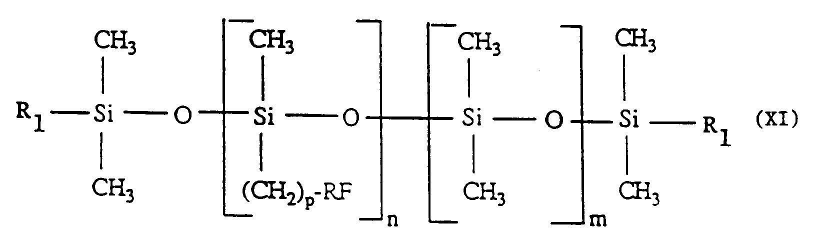 dans laquelle :
R₁ représente -CH₃, -OH ou (̵CH₂)̵ₚRF
n est un nombre entier de 1 à 300
m est un nombre entier de 0 à 150
p est un nombre entier de 0 à 6 et
RF est un radical perfluoroalkyle ayant de 1 à 9 atomes de carbone.

15. Composition selon l'une quelconque des revendications précédentes caractérisée par le fait que l'huile fluorée est présente à raison de 0,01 à 90 % et de préférence de 0,1 à 50 % en poids par rapport au poids total de la composition.

16. Composition selon l'une quelconque des revendications précédentes, caractérisée par le fait que la solution aqueuse du polymère hydrosoluble comprend en outre de 0,01 à 50 % en poids et de préférence de 0,05 à 40 % par rapport au poids total de la dispersion, d'au moins un autre polymère filmogène hydrosoluble choisi parmi :
- les dérivés de la kératine, tels que les hydrolysats de kératine et les kératines sulfoniques,
- les dérivés de chitine ou de chitosane anioniques, cationiques, amphotères ou non ioniques,
- les dérivés de cellulose, tels que l'hydroxyéthylcellulose, l'hydroxypropylcellulose, la méthylcellulose, l'éthylhydroxyéthylcellulose, la carboxyméthylcellulose, et les dérivés quaternisés de la cellulose,
- les polymères acryliques, tels que les polyacrylates et les polyméthacrylates, et les copolymères acryliques,
- la polyvinylpyrrolidone et les copolymères vinyliques tels que le copolymère de l'éther méthylvinylique et de l'anhydride maléique éventuellement monoestérifié ou monoamidifié, ou le copolymère de l'acétate de vinyle et de l'acide crotonique, et
- les polymères naturels tels que les gommes arabiques, la gomme de guar, les dérivés du xanthane et la gomme de karaya, les alginates et les carraghénates, les glycoaminoglycanes, l'acide hyaluronique et ses dérivés.

17. Composition selon l'une quelconque des revendications précédentes caractérisée par le fait que la taille des particules de la dispersion est comprise entre 0,2 et 50 µm, et de préférence entre 0,5 et 10 µm.

18. Composition selon l'une quelconque des revendications précédentes, caractérisée par le fait qu'elle renferme en outre de 0,01 à 30 % en poids par rapport au poids total de la composition d'au moins un ingrédient cosmétique choisi parmi les agents hydratants, les plastifiants, les amincissants, les agents anti-rides, les stimulants, les revitalisants, les raffermissants, les adoucissants, les épaississants, les huiles émulsionnées, les polymères non filmogènes, les conservateurs, les séquestrants, les agents anti-mousse, les parfums, les colorants, les pigments, les charges et les modificateurs de pH.

19. Composition selon l'une quelconque des revendications précédentes, caractérisée par le fait qu'elle renferme un alcool de bas poids moléculaire en une proportion de 0,01 à 20 %.

20. Composition selon l'une quelconque des revendications précédentes, caractérisée par le fait qu'elle se présente sous forme d'un gel, d'un masque, d'une crème, d'un eye-liner, d'un mascara, d'un vernis à ongles aqueux, d'une laque à l'eau, d'un gel de protection labiale ou d'un produit de coloration temporaire des cheveux.

21. Procédé de préparation d'une composition selon l'une quelconque des revendications 1 à 20, caractérisé par le fait que l'on dissout dans l'eau une quantité dudit polymère hydrosoluble comprenant de 50 à 100 % de motifs de formule (I) en une proportion telle que la viscosité ne dépasse pas environ 10.000 mPa.s et qu'ensuite, après addition éventuelle d'additifs cosmétiques ou dermatologiques, on disperse, sous vive agitation, dans la solution obtenue de 0,01 à 90 %, et de préférence de 0,1 à 50 % en poids par rapport au poids total de la composition, au moins une huile fluorée.

## Claims

1. Cosmetic or dermatological composition in the form of an oil-in-water dispersion capable of giving a composite film by evaporation of the continuous aqueous phase, characterized by the fact that it comprises at least one fluorinated oil dispersed in an aqueous solution of at least one water-soluble polymer comprising 50 to 100 mol % of units of the following formula:

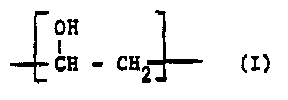

2. Composition according to Claim 1, characterized by the fact that the water-soluble polymer also comprises from 1 to 50 mol % of units of the following formula: 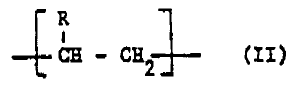 in which:
R is a hydrogen atom, a chlorine atom, a fluorine atom or represents the group 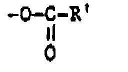 or the group OR',
R' representing an alkyl radical preferably having from 1 to 6 carbon atoms.

3. Composition according to Claim 1 or 2, characterized by the fact that the water-soluble polymer has a molecular weight of between 15,000 and 250,000, preferably between 25,000 and 200,000.

4. Composition according to any one of the preceding claims, characterized by the fact that the water-soluble polymer is chosen from: polyvinyl alcohol, polyvinyl alcohol/vinyl acetate and polyvinyl alcohol/ethylene copolymers.

5. Composition according to any one of the preceding claims, characterized by the fact that the water-soluble polymer is present in an amount of 0.5 to 40 % and preferably 0.75 to 20 % by weight relative to the total weight of the composition.

6. Composition according to any one of the preceding claims, characterized by the fact that the fluorinated oil is either an optionally functionalized carbon-containing oil whose hydrogen atoms have been partially or completely replaced by fluorine atoms, or a fluorinated silicone.

7. Composition according to Claim 6, characterized by the fast that the fluorinated oil is chosen from partially fluorinated and perfluorinated, organofluorinated hydrocarbon compounds, perfluoropolyethers and fluorohydrocarbon polyethers.

8. Composition according to Claim 7, characterized by the fact that the perfluorinated, organofluorinated hydrocarbon compounds are chosen from perfluoroalkanes, perfluorocycloalkanes, perfluoropolycycloalkanes, perfluoro(alkylcycloalkanes), perfluoroarenes and perfluorinated hydrocarbons containing at least one heteroatom.

9. Composition according to Claim 7, characterized by the fact that the partially fluorinated, organofluorinated hydrocarbon compounds correspond to the following formula:
(RF)ₓ-(A)_{y}-(RH)_{z} (III)
in which:
x may be 1, 2 or 3;
z may be 0, 1, 2 or 3;
y may be 0 or 1;
y and z not being able simultaneously to be zero, and
when z = 0, then y = 1 find x = 2 or 3;
RF represents a saturated or unsaturated, aliphatic or aromatic fluorinated radical whose chain may be linear, branched or cyclic, preferably a perfluoroalkyl radical having from 6 to 20 carbon atoms, it being possible for the chain to be optionally functionalized and/or it being possible for it to be interrupted by divalent atoms ouch as oxygen or sulphur, or trivalent atoms such as nitrogen, it being possible, in addition, for the chain to be substituted by hydrogen or other halogen atoms, on the condition that not more than one of these substituents, other than fluorine, is present for 2 carbon atoms,
RH represents a saturated or unsaturated, aliphatic or aromatic hydrocarbon residue whose chain may be linear, branched or cyclic, optionally functionalized and/or interrupted by divalent atoms such as oxygen or sulphur, or by trivalent atoms such as nitrogen,
and A represents a covalent bond when y = 0, and when y is different from 0, A represents a di- or polyvalent radical.

10. Composition according to Claim 9, characterized by the fact that the said partially fluorinated, organofluorinated hydrocarbon compounds correspond to the following formula: 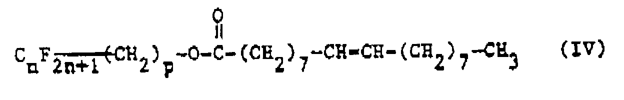 in which:
n is a number equal to 6 or 8 and
p is 1 or 2.

11. Composition according to Claim 9, characterized by the fact that the said partially fluorinated, organofluorinated hydrocarbon compounds correspond to the following formula:
R_{f}-(CH₂)ₙ-X-[C₃H₅(OH)]-(O)ₓ-Rₕ (V)
where [C₃H₅(OH)] represents 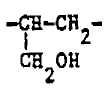 or -CH₂-CHOH-CH₂-R_{f} represents a perfluorinated C₄-C₂₀ alkyl radical or a mixture of perfluorinated C₄-C₂₀ radicals,
Rₕ represents a linear or branched C₁-C₂₂ alkyl radical or a mixture of linear or branched C₁-C₂₂ alkyl radicals or a C₆-C₁₀ aryl or C₇-C₁₅ aralkyl radical,
n is between 0 and 4,
X represents a sulphur or oxygen atom, and
x represents 0 or 1.

12. Composition according to Claim 7, characterized by the fact that the non-functionalized perfluoropolyethers are chosen from the following formulae:
(i) 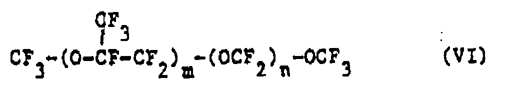 in which; m/n ranges from 5 to 40, the average molecular weight being greater than 500,
(ii)
CF₃-(O-CF₂CF₂)ₚ-(OCF₂)_{q}-OCF₃ (VII)
in which: p/q is 0.5 to 1.5, the average molecular weight being greater than 500,
(iii) 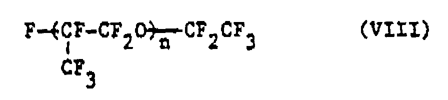 in which: n is an integer from 4 to 500, and
(iv) 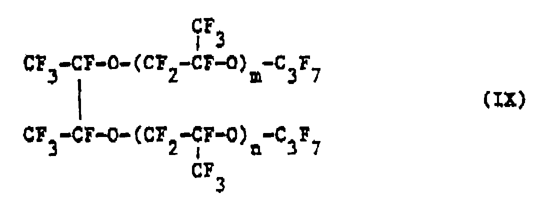 in which: n and m are integers from 0 to 3, or one of their mixtures.

13. Composition according to Claim 7, characterized by the fact that the said functionalized perfluoropolyethers correspond to the the following formula:
RCF₂-(OCF₂CF₂)ₚ-(OCF₂)_{q}- OCF₂R (X)
in which: p/q varies from 0.5 to 1.5 and
R is a -COOCH₃, -CH₂OH, -CH₂O-CH₂-CHOH-CH₂OH or -CH₂-(OCH₂-CH₂)ₜ-OH residue, where t is 1 or 2, the average molecular weight being greater than 500.

14. Composition according to Claim 6, characterized by the fact that the fluorinated silicone corresponds to the formula (XI): 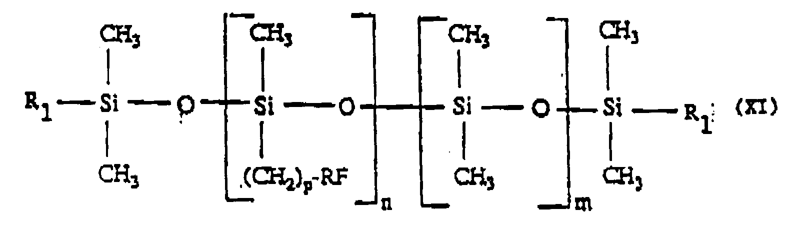 in which:
R₁ represents -CH₃, -OH or (̵CH₂)̵ₚRF,
n is an integer from 1 to 300,
m is an integer from 0 to 150,
p is an integer from 0 to 6 and
RF is a perfluoroalkyl radical having 1 to 9 carbon atoms.

15. Composition according to any one of the preceding claims, characterized by the fact that the fluorinated oil is present in an amount of 0.01 to 90 %, and preferably 0.1 to 50 % by weight relative to the total weight of the composition.

16. Composition according to any one of the preceding claims, characterized by the fact that the aqueous solution of the water-soluble polymer contains, in addition, 0.01 to 50 % by weight and preferably from 0.05 to 40 % relative to the total weight of the dispersion of at least one other water-soluble film-forming polymer chosen from:
- keratin derivatives such as keratin hydrolysates and sulphonic keratins,
- anionic, cationic, amphoteric or non-ionic chitin or chitosan derivatives,
- cellulose derivatives such as hydroxyethyl cellulose, hydroxypropyl cellulose, methylcellulose, ethyl hydroxyethyl cellulose, carboxymethylcellulose and the quaternized derivatives of cellulose,
- acrylic polymers such as polyacrylates and polymethacrylates, as well as acrylic copolymers,
- polyvinylpyrrolidone and the vinyl copolymers, such as methyl vinyl ether and maleic anhydride copolymer optionally monoesterified or monoamidated, or vinyl acetate and crotonic acid copolymer, and
- natural polymers such as gum Arabic, guar gum, xanthan derivatives and karaya gum, alginates and carrageenans, glycoaminoglycans, hyaluronic acid and its derivatives.

17. Composition according to any one of the preceding claims, characterized by the fact that the size of the particles of the dispersion is between 0.2 and 50 µm, and preferably between 0.5 and 10 µm.

18. Composition according to any one of the preceding claims, characterized by the fact that it contains, in addition, 0.01 to 30 % by weight relative to the total weight of the composition of at least one cosmetic ingredient chosen from moisturizing agents, plasticizers, slimming agents, antiwrinkle agents, stimulants, revitalizing agents, toning agents, emollients, thickeners, emulsified oils, non-film-forming polymers, preservatives, sequestrants, antifoaming agents, perfumes, colorants, pigments, fillers and pH modifiers.

19. Composition according to any one of the preceding claims, characterized by the fact that it contains a low-molecular weight alcohol in a proportion of 0.01 to 20 %.

20. Composition according to any one of the preceding claims, characterized by the fact that it is provided in the form of a gel, a face pack, a cream, an eye-liner, a mascara, an aqueous nail varnish, a water-containing lacquer, a lip-protesting gel or a product for temporarily dyeing the hair.

21. Process for preparing a composition according to any one of Claims 1 to 20, characterized by the fact that a quantity of the said water-soluble polymer containing 50 to 100 % of units of formula (I) is dissolved in water in a proportion such that the viscosity does not exceed about 10,000 mPa.s, and that next, after the optional addition of cosmetic or dermatological additives, 0.01 to 90 %, and preferably 0.1 to 50 % by weight relative to the total weight of the composition of at least one fluorinated oil is dispersed, with vigorous stirring, in the solution obtained.

## Patentansprüche

1. Kosmetische oder dermatologische Zubereitung in Form einer Öl-in-Wasser-Dispersion, die dazu befähigt ist, durch Verdampfung der wäßrigen kontinuierlichen Phase einen Verbundfilm zu ergeben, wobei die Zubereitung dadurch gekennzeichnet ist, daß sie mindestens ein in einer Lösung von mindestens einem wasserlöslichen Polymer dispergiertes, fluoriertes Öl enthält, das 50 bis 100 Mol-% an Einheiten gemäß der folgenden Formel 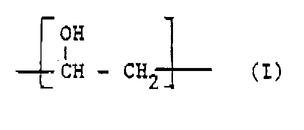 enthält.

2. Zubereitung nach Anspruch 1, dadurch gekennzeichnet, daß das wasserlösliche Polymer zusätzlich noch 1 bis 50 Mol-% der folgenden Formel 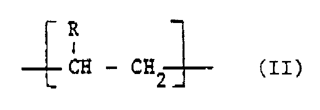 enthält, worin
R ein Wasserstoffatom, ein Chloratom, ein Fluoratom oder den Rest 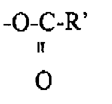 oder den Rest OR' bedeutet, wobei R' einen Alkylrest mit vorzugsweise 1 bis 6 Kohlenstoffatomen darstellt.

3. Zubereitung nach Anspruch 1 oder 2, dadurch gekennzeichnet, daß das wasserlösliche Polymer ein Molekulargewicht zwischen 15.000 und 250.000, vorzugsweise zwischen 25.000 und 200.000 aufweist.

4. Zubereitung nach einem der vorstehenden Ansprüche, dadurch gekennzeichnet, daß das wasserlösliche Polymer unter Polyvinylalkohol, Polyvinylalkohol/Vinylacetat-Copolymeren und Polyvinylalkohol/Ethylen ausgewählt ist.

5. Zubereitung nach einem der vorstehenden Ansprüche, dadurch gekennzeichnet, daß das wasserlösliche Polymer in einem Gewichtsverhältnis von 0,5 bis 40 %, vorzugsweise von 0,75 bis 20 Gew.-% in bezug auf das Gesamtgewicht der Zusammensetzung vorhanden ist.

6. Zubereitung nach einem der vorstehenden Ansprüche, dadurch gekennzeichnet, daß das fluorierte Öl gegebenenfalls ein mit funktionellen Gruppen versehenes Öl mit Kohlenstoffatomen darstellt, bei dem die Wasserstoffatome teilweise oder vollständig durch Fluoratome ersetzt sind, oder daß das fluorierte Öl ein fluoriertes Silikon darstellt,

7. Zubereitung nach Anspruch 6, dadurch gekennzeichnet, daß das fluorierte Öl unter teilweise fluorierten und perfluorierten Organofluorkohlenwasserstoffverbindungen, Perfluorpolyethern sowie Fluorkohlenwasserstoffpolyethern ausgewählt ist.

8. Zubereitung nach Anspruch 7, dadurch gekennzeichnet, daß die perfluorierten Organofluorkohlenwasserstoffe aus Perfluoralkanen, Perfluorcycloalkanen. Perfluorpolycycloalkanen, Perfluor(alkylcycloalkanen), Perfluorarenen und perfluorierten Kohlenwasserstoffen mit mindestens einen, Heteroatom ausgewählt sind.

9. Zubereitung nach Anspruch 7, dadurch gekennzeichnet, daß die teilweise fluorierten Organofluorkohlenwasserstoffverbindungen der folgenden Formel entsprechen:
(RF)ₓ-(A)_{y}-(RH)_{z} (III)
worin jeweils:
x 1, 2 oder 3
z 0,1, 2 oder 3,
y 0 oder 1 bedeuten können,
wobei z und y nicht gleichzeitig 0 bedeuten können, wobei noch die Maßgabe gilt, daß in dem Falle, daß z = 0, dann y = 1 und x = 2 oder 3 sind;
RF einen fluorierten, gegebenenfalls ungesättigten aromatischen oder aliphatischen Rest bedeutet, der gerad- oder verzweigtkettig oder cyclisch sein kann, vorzugsweise einen Perfluoralkylrest mit 6 bis 20 Kohlenstoffatomen darstellt, wobei die Kohlenstoffkette gegebenenfalls funktionelle Gruppen enthalten und/oder durch zweiwertige Atome, beispielsweise Schwefel oder Sauerstoff oder auch durch dreiwertige Atome wie Stickstoff unterbrochen sein kann, wobei die Kohlenstoffkette zusätzlich noch durch Wasserstoffatome oder weitere Atome in Form von Halogenatomen mit der Maßgabe substituiert sein kann, daß nicht mehr als einer dieser von Fluor verschiedenen Substituenten für 2 Kohlenstoffatome vorhanden sein kann, und
RH einen gegebenenfalls ungesättigten aliphatischen oder aromatischen Kohlenwasserstoffrest bedeutet, der gerad- oder verzweigtkettig oder cyclisch und gegebenenfalls mit funktionellen Gruppen versehen sein kann sowie/oder durch zweiwertige Atome wie z.B. Sauerstoff oder Schwefel wie auch durch dreiwertige Atome, wie auch z.B. Stickstoff, unterbrochen sein kann, und
A eine kovalente Bindung für den Fall bedeutet, daß y = 0,
oder für den Fall, daß y von 0 verschieden ist, A einen zwei- oder mehrwertigen Rest darstellt.

10. Zubereitung nach Anspruch 9, dadurch gekennzeichnet, daß die teilweise fluorierten Organofluorkohlenwasserstoffverbindungen der folgenden Formel entsprechen: 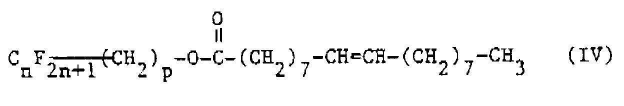 worin
n eine Zahl gleich 6 oder 8 sowie
p die Zahl 1 oder 2 bedeuten,

11. Zubereitung nach Anspruch 9, dadurch gekennzeichnet, daß die teilweise fluorierten Organofluorkohlenwasserstoffverbindungen der folgenden Formel entsprechen:
R_{f}-(CH₂)ₙ-X-[C₃H₅(OH)]-(O)ₓ-Rₕ (V)
oder [C₃H₅(OH)] in Form von 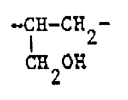 ou -CH₂-CHOH-CH₂-vorliegt, wobei R_{f} einen perfluorierten C₄-C₂₀ Alkylrest oder eine Mischung aus perfluorierten C₄-C₂₀-Resten bedeutet, Rₕ einen gerad- oder verzweigtkettigen C₁-C₂₂-Alkylrest, ein Gemisch aus linearen oder verzweigten C₁-C₂₂-Alkylresten, einen C₆-C₁₀-Arylrest oder einen C₇₋₁₅ Alkylrest darstellt, wobei n zwischen 0 und 4 ist, X ein Schwefel- oder Sauerstoffatom darstellt und x die Zahl 0 oder 1 bedeutet,

12. Zubereitung nach Anspruch 7, dadurch gekennzeichnet, daß die nicht mit funktionellen Gruppen versehenen Perfluorpolyether unter den folgenden Formeln ausgewählt sind:
(i) 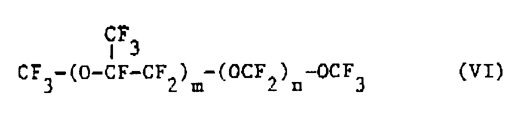 worin
m/n zwischen 5 und 40 variert, wobei das mittlere Molekulargewicht mehjr als 500 beträgt,
(ii)
CF₃-(O-CF₂CF₂)ₚ-(OCF₂)_{q}-OCF₃ (VII)
worin
p/q zwischen 0,5 und 1 variiert, wobei das durchschnittliche Molekulargewichtmehr als 500 beträgt,
(iii) 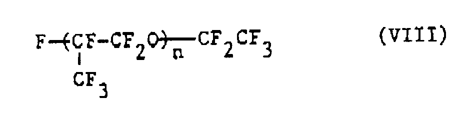 worin
n eine ganze Zahl von 4 bis 500 ist sowie
(iv) 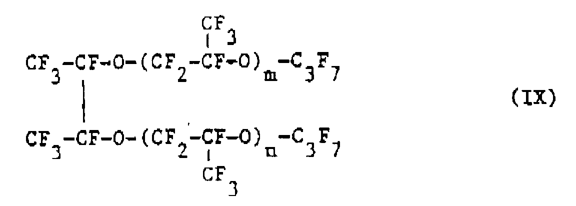 worin
n und m ganze Zahlen von 0 bis 3 bedeuten,
oder eines ihrer Gemische.

13. Zubereitung nach Anspruch 7, dadurch gekennzeichnet, daß die mit funktionellen Gruppen substituierte Perfluorpolyether der folgenden Formel entsprechen:
RCF₂-(OCF₂CF₂)ₚ-(OCF₂)_{q}- OCF₂R (X)
worin
p/q zwischen 0,5 und 1,5 variiert, wobei
R eine Gruppe -COOCH₃, -CH₂OH, -CH₂O-CH₂CHOH-CH₂OH oder -CH₂-(OCH₂-CH₂)ₜ-OH bedeutet, worin
t die Zahl 1 oder 2 darstellt, wobei das durchschnittliche Molekulargewicht höher als 500 ist.

14. Zubereitung nach Anspruch 6, dadurch gekennzeichnet, daß das fluorierte Silikon der Formel (XI) entspricht: 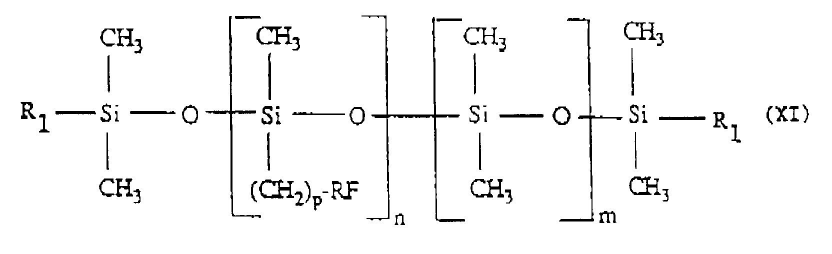 worin
R₁ -CH₃, -OH oder (CH₂)ₚ RF bedeutet,
n eine ganze Zahl von 1 bis 300,
m eine ganze Zahl von 0 bis 150 und
p eine ganze Zahl von 0 bis 6 bedeuten, wobei
RF einen Perfluoralkylrest mit 1 bis 9 Kohlenstoffatomen darstellt.

15. Zubereitung nach einem der vorstehenden Ansprüche, dadurch gekennzeichnet, daß das fluorierte Öl in einem Gewichtsverhältnis von 0,01 bis 90 Gew.-%, vorzugsweise von 0,1 bis 50 Gew.-% in bezug auf das Gesamtgewicht der Zusammensetzung vorhanden ist.

16. Zubereitung nach einem der vorstehenden Ansprüche, dadurch gekennzeichnet, daß die wässrige Lösung des wasserlöslichen Polymeren zusätzlich noch 0,01 bis 50 Gew.-%, vorzugsweise 0,05 bis 40 Gew.-% in bezug auf das Gesamtgewicht der Dispersion, sowie mindestens ein weiteres wasserlösliches filmbildendes Polymer enthält, das unter den folgenden Verbindungen ausgewählt ist:
- Keratinderivate, beispielsweise Hydrolysate von Keratin und sulfonierten Keratinen,
- anionische, kationische, amphotere oder nichtionische Chitin- oder Chitosanderivate,
- Cellulosederivate, wie z.B. Hydroxyethylcellulose, Hydroxypropylcellulose, Methylcellulose, Ethylhydroxyethylcellulose, Carboxymethylcellulose und quaternisierte Cellulosederivate,
- Acrylpolymere, wie z.B. Polyacrylate und Polymethacrylate sowie Acrylcopolymere,
- Polyvinylpyrrolidon und Vinylcopolymere, wie z.B. das Copolymer aus Methylvinylmaleinsäureanhydridester, der gegebenenfalls in Form eines Monoesters oder Monoamids vorliegt, oder das Crotonsäurevinylacetatcopolymer und
- natürliche Copolymere, wie z.B. Gummiarabicumsorten, Guargummi, Xanthanderivate oder Karayagummi, Alginate und Carragheenate, Glycosaminoglykane, Hyaluronsäure und deren Derivate.

17. Zubereitung nach einem der vorstehenden Ansprüche, dadurch gekennzeichnet, daß die Teilchengröße der Dispersion zwischen 0,2 und 50 µm, vorzugsweise zwischen 0,5 und 10 µm beträgt.

18. Zubereitung nach einem der vorstehenden Ansprüche, dadurch gekennzeichnet, daß sie zusätzlich noch 0,01 bis 30 Gew.-% in bezug auf das Gesamtgewicht der Zusammensetzung mindestens eines kosmetischen Hilfsstoffs enthält, der unter Hydratisierungsmitteln, Weichmachern, Schlankmachern, Mitteln gegen Falten, Stimulantien, Revitalisierungsmitteln, Stärkungsmitteln, Erweichungsmitteln, Verdickungsmitteln, emulgierten Ölen, nicht-filmbildenden Polymeren, Konservierungsmitteln, Komplexiermitteln, Antischäumungsmitteln, Duftstoffen, Farbstoffen, Pigmenten Trägerstoffen und Mitteln zur Regulierung des PH-Wertes ausgewählt ist.

19. Zubereitung nach einem der vorstehenden Ansprüche, dadurch gekennzeichnet, daß sie einen Alkohol mit niedrigem Molekulargewicht in einem Verhältnis von 0,01 bis 20 % miteinschließt.

20. Zubereitung nach einem der vorstehenden Ansprüche, dadurch gekennzeichnet, daß sie in Form eines Gels, einer Maske, einer Creme, eines Augenbrauenstiftes, einer Wimperntusche, eines wäßrigen Nagellacks, eines Wasserlacks, eines Lipperschutzgeles oder eines Produkts zum vorübergehenden Färben der Haare vorliegt.

21. Verfahren zur Herstellung einer Zubereitung nach einem der Ansprüche 1 bis 20, dadurch gekennzeichnet, daß eine Menge an wasserlöslichem Polymeren mit 50 bis 100 % an Einheiten gemäß der Formel (I) in einem Verhältnis im Wasser aufgelöst wird, das so bemessen wird, daß die Visokosität einen Wert von etwa 10.000 mPa . s nicht überschreitet und im Anschluß daran nach der eventuellen Zugabe von kosmetischen oder dermatologischen Hilfsstoffen unter heftigem Rühren in der erhaltenen Lösung 0,01 bis 90 Gew.-%, vorzugsweise 0,1 bis 50 Gew.-% in bezug auf das Gesamtgewicht der Zusammensetzung mindestens ein fluoriertes Öl dispergiert wird.
